# EUROPEAN PATENT SPECIFICATION

(11) **EP 1 326 609 B1**
(45) Date of publication and mention of the grant of the patent: **17.08.2005**
(21) Application number: 01978022.0
(22) Date of filing: 18.10.2001
(51) Int. Cl.: A61K 31/44, A61K 31/4439, A61K 9/28, A61K 9/20

(54) **STABLE ORAL FORMULATION CONTAINING BENZIMIDAZOLE DERIVATIVE**
STABILE ORALE ZUSAMMENSETZUNG ENTHALTEND BENZIMIDAZOL DERIVATE
FORMULATION ORALE STABLE A BASE DE DERIVE DE BENZIMIDAZOLE

(30) Priority: 20.10.2000 WO PCT/BE00/00126
(43) Date of publication of application: 16.07.2003
(73) Proprietor: Galephar M/F, 6900 Marche-en-Famenne (BE)
(72) Inventor: VANDERBIST, Francis, B-1650 Beersel (BE); SERENO, Antonio, B-1820 Melsbroek (BE); BAUDIER, Philippe, B-1180 Uccle (BE); DEBOECK, Arthur, Gurabo, Puerto Rico 00778 (US)
(74) Representative: Powis de Tenbossche, Roland
(86) International application number: PCT/BE2001/000184
(87) International publication number: WO 2002/032425

(56) References cited:
- EP-A- 1 004 305
- WO-A-99/32091
- DE-A- 19 918 434
- US-A- 5 708 017

## Description

### Field of the invention

The present invention relates to a stable, pharmaceutically oral dosage form of a benzimidazole derivative as well as to an advantageous and economical process for manufacturing the same.

### Description of the background

Benzimidazole compounds are very effective drugs for the treatment of gastric and duodenal ulcers, gastroesophageal reflux disease, severe erosive esophagitis, Zollinger-Ellison syndrome and H pylori eradication. However, it is well known that these compounds have poor stability. In the solid state they are susceptible to heat, moisture and light, and in aqueous solution or suspension their stability decreases with decreasing pH. The degradation of these compounds is catalyzed by acidic reacting compounds. The main benzimidazole derivatives used in therapeutics at the moment are omeprazole, lansoprazole, pantoprazole and rabeprazole.
Omeprazole or 5-methoxy-2 (((4-methoxy-3,5-dimethyl-2-pyridinyl) methyl)sulfinyl)-1H-benzimidazole is a useful and very widely used treatment of gastric and duodenal ulcer, erosive esophagitis and gastroesophagal reflux disease. Omeprazole acts by inhibiting gastric acid secretion. The usual daily dosage is from 10 to 100 mg of omeprazole in one dose.
The formulation of omeprazole must be protected from gastric fluids since it is rapidly chemically degraded at acidic pH. Consequently, omeprazole is usually released in the proximal parts of the small intestine where it is rapidly absorbed. The absolute bioavailability of omeprazole with doses of 20 to 40 mg/day is approximately 30% to 40%.

Different oral compositions of omeprazole and other benzimidazole derivatives have been described in the past. The US patent 4786505 describes a pharmaceutical preparation containing an acid labile compound together with an alkaline reacting compound and together with an alkaline compound as the core material. This patent also described one or more subcoating layers and an enteric coating as well as a process for the preparation thereof.
The US Patent 5232706 is quite close to the one mentioned hereinabove. It describes a preparation comprising a nucleus formed by a mixture of omeprazole with a basic compound. The nucleus has two coatings. The first is formed by an enteric coating.
The US Patent 5385739 relates to a stable formulation of omeprazole microgranules containing a neutral core consisting of sugar and starch, characterized in that it contains an active layer consisting of a dilution of omeprazole in mannitol in substantially equal amounts. It also relates to a process for producing such formulations.
The US Patent 5690960 relates to a new oral pharmaceutical formulation containing a novel physical form of a magnesium salt of omeprazole, a method for the manufacture of such a formulation.
EP 1 004 305 relates to chemically stabilized preparations of benzimidazole-type compounds. These compositions comprise the benzimidazole-type compounds or alkali metal salts thereof together with at least one substance selected from among sodium carbonate, potassium carbonate, sodium hydroxide, potassium hydroxide, aminoalkyl methacrylate copolymer E , arginine aspartate, hydroxypropyl cellulose and crospovidone.
Finally, the US Patent 5817338 describes a new pharmaceutical multiple unit tabletted dosage form containing omeprazole, a method for the manufacture of such formulation, and the use of such formulation in medicine.

Benzimidazole derivatives degrade very rapidly in water solutions at low pH values. The rate of degradation of omeprazole, for instance, proceeds with a half-life of less than 10 minutes at pH values below 4, At pH 6.5, the half-life of degradation is 18 hours; at pH 11 about 300 days. But omeprazole is susceptible to degradation not only in an acidic environment but also under the influence of temperature, humidity, organic solvents and oxygen. Degradation of omeprazole (and of other benzimidazole derivatives) is known to give decomposition products that are highly colored. Consequently, inappropriate conditions of handling of the product will cause discoloration even at small levels of degradations.
The galenic formulation and the manufacturing process should therefore be carefully optimized to guarantee the stability of the composition through the entire shelf-life of the drug medicine.

### Brief description of the invention

An object of the present invention is to provide a stable oral composition of a benzimidazole derivative and a process thereof. The new dosage form is characterized as follows: the benzimidazole derivative is formulated in the form of an enteric coated tablet. The core tablet contains at least, in addition to the active ingredient, one lipophilic antioxidant agent. An insulating coating layer may advantageously be applied on the core tablets before the enteric coating.

The invention relates thus to an enteric coated tablet formulation containing at least one benzimidazole derivative, said formulation comprising:
- a core containing at least one benzimidazole derivative and at least one lipophilic antioxidant,
- an enteric envelope protecting the core at a pH below 5.

The core of the present invention is a tablet.
Preferably, the invention relates to an enteric coated tablet containing at least one benzimidazole derivative. The tablet of the invention comprises a core containing at least one benzimidazole derivative and at least one lipophilic antioxidant, said core being provided with at least one enteric coating layer.

According to a preferred embodiment, the tablet of the invention comprises:
- a core containing at least said benzimidazole derivative and at least one lipophilic antioxidant;
- an enteric coating layer, and
- a pre-coating layer or insulating layer extending between the core and the enteric coating layer.

Advantageously, the core comprises at least one tabletting excipient and one lipophilic antioxidant. Preferably, the core tablet is manufactured using a direct compression process. Alternatively, a wet granulation process may be used.
In this case, at least a part of the lipophilic antioxidant is adsorbed on a tabletting agent or granulated with a tabletting agent.
Preferably, the enteric coating or envelope is substantially free of benzimidazole derivative, and is most preferably free of benzimidazole derivative. A pre-coating layer or an insulating layer may advantageously be applied on the core tablet before the enteric coating.
The pre-coating layer or insulating layer is also advantageously substantially free of benzimidazole derivative.
According to a detail of an embodiment, the core comprises at least a tabletting excipient selected among the group consisting of microcrystalline cellulose, cellulose derivatives, lactose, mannitol, mono or disaccharide, and mixtures thereof, blended with at least one lipophilic antioxidant is attached.

Advantageously, at least one lipophilic antioxidant agent is selected from the group consisting of derivatives of vitamin E (α-tocopherol) or vitamin C (ascorbic acid), Butylhydroxyamide (BHA), butylhydroxytoluene (BHT), or propyl gallate, lipoïc acid and mixtures thereof. Preferably, substantially all the lipophilic antioxidant agent(s) present in the core is (are) selected from said groups.
Preferably, the lipophilic antioxidant comprises at least ascorbyl palmitate and is most preferably ascorbyl palmitate.
Advantageously, the lipophilic antioxidants chosen are solid at ambient temperature like BHA, BHT, propyl gallate or ascorbyl palmitate in order to allow a direct compression process for the manufacturing of the tablet. If the lipophilic antioxidant is liquid (like vitamin E derivatives or lipoïc acid), the manufacturing of the tablet involves a granulation step between the liquid antioxidant and one tabletting agent. This granulation step requires a drying step and consequently makes the manufacturing process of the present invention longer, more complicated and more expensive.
The pre-coating layer or the insulating layer comprises advantageously at least a polymer selected from the group consisting of povidone, derivatives of povidone, derivatives of cellulose, and mixtures thereof. Preferably, said polymer(s) forms at least 50% by weight (most preferably at least 75% by weight, for example substantially completely) of the dry pre-coating layer or insulating layer. The pre-coating solution is advantageously water-free.

The enteric layer or envelope comprises advantageously at least one cellulosic polymer or cellulosic derivative. Preferably, the dry enteric layer or envelope comprises from 20 to 70% by weight (most preferably from 30 to 60% by weight, especially about 50% by weight) of cellulosic polymer and cellulosic derivative. According to a preferred embodiment, the enteric layer or envelope comprises at least hypromellose phthalate as cellulosic derivative and/or at least an acrylic/methacrylic polymer or copolymer, preferably a methacrylic acid copolymer.

The benzimidazole derivative is advantageously selected from the group consisting of benzimidazole derivatives inhibiting the proton pump, pantoprazole, lansoprazole, omeprazole, rabeprazole and mixtures thereof. According to a specific embodiment, the benzimidazole derivative is omeprazole.

According to a possible embodiment, the tablet of the invention or the capsule of the invention contains from 5 to 80 mg omeprazole. According to another possible embodiment, the tablet of the invention or the capsule of the invention contains from 5 to 60 mg of lansoprazole.

The invention also relates to a process for the preparation of a formulation of the invention, in which the core is prepared by direct compression or alternatively in which the manufacturign of the core involves the granulation of the lipophilic antioxidant with at least one tabletting excipient, and in which the core is provided with at least an enteric layer or envelope.
Advantageously, the process is to blend all the excipients contained in the core of the present invention in one single step and to manufacture the tablets by direct compression.
The core has advantageously the form of a tablet, which is provided with a pre-coating and an enteric coating using the pan-coating technology or the fluid bed technology.

### Brief description of the drawing

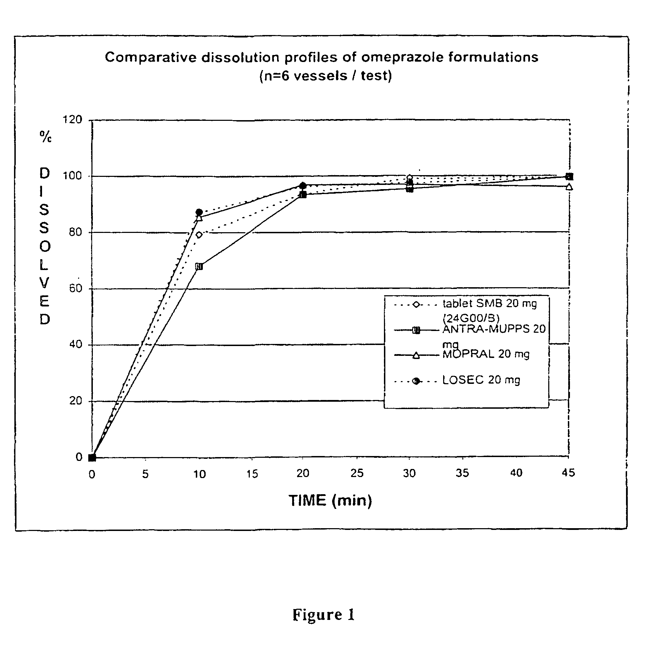

Figure 1 gives the dissolution profiles of omeprazole formulation of the invention (tablet SMB 20 mg), as well as of marketed omeprazole formulations.
Conditions of the tests: paddle apparatus, 75 rpm, pH = 7.5, 37°C.

### Description of examples of the invention

A preferred embodiment of the invention is a stable formulation of omeprazole or of another benzimidazole derivative under the form of a pharmaceutical coated tablet.

The tablet comprises a core which contains, in addition to several excipients used in the manufacturing of pharmaceutical tablets, a lipophilic antioxidant derivative.

The tablet may be manufactured using the direct compression technology if the lipophilic antioxidant chosen is a powder (ascorbyl palmitate for instance). If the lipophilic antioxidant chosen is a liquid (vitamin E derivatives), it is needed to first granulate or adsorbate the said lipophilic excipient together with another tabletting excipient, preferably with microcrystalline cellulose.

This adsorbate is then mixed with the active ingredient and the other tabletting excipients. The whole blend is tabletted by a direct compression process.

The adsorbate mentioned hereinabove is formed by melting the lipophilic antioxidant derivative and adding it in the liquid form to a classical tabletting excipient in a planetary mixer. The antioxidant derivative solidifies when put in contact with the tabletting excipient.

It has been found that by using the lipophilic antioxidant, in the form of a dry blend or the antioxidant adsorbate, it was possible to prepare formulation having an excellent stability. The core of the tablet so manufactured is coated as follows: first with an insulating layer and then with an enteric coating layer.

The direct coating of the tablets with the enteric layer was prevented in the preferred example, so as to avoid possible degradation of the active ingredient due to the presence of acidic groups in the enteric polymer. Therefore, a neutral coating layer is advantageously applied on the core tablets before the application of the enteric coating.

The insulating coating layer of these examples contains at least one water soluble polymer as, for example, povidone or hypromellose. Povidone is the preferred excipient for the insulating layer because this polymer is soluble in absolute alcohol while the cellulosic derivatives need traces of water to be completely soluble. And it is well known that the presence of water, even in traces, is able to accelerate/provoke a chemical degradation of benzimidazole derivatives.

The enteric coating polymer may be a derivative of cellulose (cellulose acetophthalate, hypromellose phthalate) or a derivative of an acrylic polymer (methacrylate acid copolymer).

The preferred enteric polymer must be able to protect the formulation at acidic pH corresponding to the transit in the stomach (pH comprised between 1 and 5) and to release the active ingredient rapidly once the formulation arrives in small intestine. Therefore, hypromellose phthalate (HP50®, Shinetzu) is the preferred polymer for this purpose since it has the properties to be soluble at pH>5.0.

Several formulations for the core of the example of tablets, the insulating coating layer and the enteric coating layer are given hereinbelow. Those formulation are not limitative and are only destinated to further describe the invention.

The formulations A to N give different formulations of the core tablet, pre-coating and enteric coating, corresponding to the present invention

| Formulation of the core tablet | | | | | | |
|---|---|---|---|---|---|---|
| | Composition in mg / tablet | | | | | |
| Ingredient | A | B | C | D | E | F |
| OMEPRAZOLE | 10 | 10 | 10 | 10 | 10 | 10 |
| Vitamine E TPGS | 10 | | | | | |
| Ascorbyl palmitate | | | | | 2 | |
| Butylhydroxyanisole | | | | | | 0.01 |
| Microcrystalline Cellulose | 16.6 | 16.6 | 16.6 | 16.6 | 16.6 | 16.6 |
| Crospovidone | 8.5 | 8.5 | | | 8.5 | 8.5 |
| Lactose | 104 | 114 | | 114 | 114 | 114 |
| Mannitol | | | 122.5 | 25.1 | | |
| Mg stearate | 1 | 1 | 1 | 1 | 1 | 1 |

| Coating isolation or pre-coating (mg of dry matter applied on a tablet) | | | | |
|---|---|---|---|---|
| Ingredient | G | H | I | J |
| Povidone | 7.5 | | 15 | |
| HPMC | | 7.5 | | 10 |
| HPMC : hydroxy propyl methyl cellulose | | | | |

The pre-coating was applied by using a solution of Povidone or HPMC, said solution containing preferably absolute ethanol as solvent or alternatively an hydro-ethanolic mixture.

| **Enteric coating (mg of dry matter applied on a tablet)** | | | | |
|---|---|---|---|---|
| Formulation Composition mg of the enteric coating | K | L | M | N |
| Eudragit (Methacrylic Acid Copolymer) L 30D - 55 | | | 7.3 (dry) | 7.3 (dry) |
| HP 50 (Hydroxypropyl Methylcellulose phthalate | 7.3 | 7.3 | | |
| Talc | 4.445 | 4.445 | 4.445 | 4.445 |
| Povidone | | | 1.818 | 1.818 |
| Triacetine | 1.836 | | | |
| Triethyl citrate | | | 1.836 | |
| Diethyl phthalate | | 1.836 | | |
| Polyethylene glycol | | | | 1.836 |
| Red iron oxide | 1.43 | 1.43 | 1.43 | 1.43 |

The enteric coating was applied by using a solution containing the different compounds listed in the above table, and a hydro-ethanolic mixture, the weight ratio compounds listed in the table/ hydro-ethanolic mixture being 15/85.

The excellent stability of omeprazole formulation of the invention containing a lipophilic antioxidant agent was demonstrated by comparing the stability of enteric coated tablets with and without an antioxidant agent.

In order to assess the influence of the presence of a lipophilic antioxidant agent in the the core tablet on the stability, different formulations (with and without lipophilic antioxidant agent) of tablet have been manufactured and all the tablets were coated with the same pre-coating and enteric coating film.

| | mg /tablet | | | | |
|---|---|---|---|---|---|
| Ingredient | 1 | 2 | 3 | 4 | 5 |
| OMEPRAZOLE | 10 | 10 | 10 | 10 | 10 |
| Vitamin E | 0 | 10 | 0 | 0 | 0 |
| Vitamin E TPGS | 10 | 0 | 0 | 0 | 0 |
| Ascorbyl palmitate | 0 | 0 | 0 | 2 | 0 |
| BHA | 0 | 0 | 0 | 0 | 0.02 |
| Microcrystalline cellulose | 16.6 | 16.6 | 16.6 | 16.6 | 16.6 |
| Crospovidone | 8.50 | 8.50 | 8.5 | 8.5 | 8.5 |
| Monohydrate lactose | 104 | 104 | 104 | 104 | 104 |
| Magnesium stearate | 1.00 | 1.00 | 1.00 | 1.00 | 1.00 |

| **Pre-coating** | | | | | |
|---|---|---|---|---|---|
| | mg / tablet | | | | |
| Ingredient | 1 | 2 | 3 | 4 | 5 |
| POVIDONE | 6.10 | 6.10 | 6.10 | 6.10 | 6.10 |

| **Enteric coating composition** | | | | | |
|---|---|---|---|---|---|
| Ingredient | 1 | 2 | 3 | 4 | 5 |
| Hypromellose phthalate | 5.60 | 5.60 | 5.60 | 5.60 | 5.60 |
| Talc | 3.40 | 3.40 | 3.40 | 3.40 | 3.40 |
| Glycerol triacetate | 2.80 | 2.80 | 2.80 | 2.80 | 2.80 |

All the tablets were packaged in high density polyethylene bottles containing a dessicant caspule (1 gram of silicagel) and put in stability at 40°C / 75 % RH.

The stability were assessed by observing the apparition of a coloration in the tablets.. This coloration corresponds to the formation of degradation products of omeprazole and appears even at very low levels of degradation (< 0.5 %).

After storing for 3 months the different compositions at 40°C/75% RH, the following observations have been made.

The formulation 3, i.e. the tablet containing no antioxidant agent showed a clear instability already after 1 month. Indeed, the tablet developed an intense violet coloration (characteristic to a degradation of omeprazole). After 3 months, the tablets were brown.

The formulation 2, i.e. the tablet containing α-tocopherol as antioxidant agent, was more stable than formulation 3 since after 1 month of storage, only a slight yellow coloration appeared on the tablet but a significant violet coloration appears after 3 months.

The formulation 1, i.e. the tablet containing Vitamin E polyethylene glycol succinate (Vitamin E TPGS) as antioxidant agent, had a better stability than that of formulation 2 and 3, since the tablet was still completely white atter 1 month of storage at 40°C/75% RH. But, after 3 months, formulation 1 showed also a slight apparition of a yellow coloration.
Formulation 4 containing ascorbyl palmitate as antioxidant gave the best stability results since no apparition of colour are observed on the tablets after 3 months at 40°C / 75%.
On the other hand, the formulation 5 , containing a non lipophilic antioxidant (ascorbic acid) did not show any improvement in term of stability in comparison with formulation 3 without antioxidant.
In summary the efficacy of the various antioxidant tested with omeprazole was : ascorbyl palmitate > BHA > Vitamine E TPGS > ascorbic acid = no antioxidant

The same tendency was observed with another benzimidazole derivative, lansoprazole, for which a formulation containing ascorbyl palmitate as antioxidant significantly improves the stability of an enteric coated tablet in comparison with an enteric tablet containing no lipophilic antioxidant. A subject matter of the invention is thus also a pharmaceutical composition (preferably for oral administration) comprising a benzimidazole derivative (preferably omeprazole and/or lansoprazole) and at least an antioxidant selected from the group consisting of ascorbyl palmitate, BHA and mixtures thereof. Still a further subject matter of the invention is a pharmaceutical composition (preferably for oral administration) comprising a benzimidazole derivative (preferably omeprazole and/or lansoprazole) and at least ascorbyl palmitate.

For showing the usefulness of the pre-coating (or insulating coating) layer, the stability of a formulation of enteric tablet (formulation 4) was compared with the same formulation but without pre-coating

The formulation 4 containing the pre-coating layer has given a product white at the end of the manufacturing process, while the formulation 4 without the pre-coating layer shows the apparition of violet spots on the omeprazole tablets. It is thought that the violet spots are due to (i) the acidic groups contained in the enteric coating layer which are able to react with omeprazole on the surface of the tablet and/or (ii) to the water contained in the enteric coating solution, said water being able to provoke and/or accelerate the degradation of omeprazole present on the surface of the tablet.

Therefore, it is thought that the insulating / pre-coating layer is useful in the present invention for protecting the omeprazole molecules located at the surface of the core tablets. The coating suspension or solution used for said pre-coating contains preferably no water (use of absolute alcohol as solvent for preparing the coating solution or suspension).

Hereinbelow is described an example of manufacturing process of a formulation of the invention, in the form of enteric coated tablets.

### STEP 0

Control of the cleanliness of premises, material and equipment

### STEP 1 : Weighing

Individual weighing of raw materials

### STEP 2 : Pre-Blending (not necessary if the lipophilic antioxidant is a solid)

### Equipment

Planetary mixer

### Operation

lipophilic antioxidant is heated until it becomes liquid. It is then adsorbed onto Microcrystalline Cellulose by a mixing operation.

If the lipophilic antioxidant chosen is a powder, no pre-blending is needed.

### STEP 3: Blending

### Equipment

Planetary mixer

### Operation

Introduce in the mixer the adsorbed lipophilic antioxidant, crospovidone, lactose, magnesium stearate and omeprazole.
Homogenise.

### STEP 4: Tabletting

### Equipment

Automatic tabletting machine type Courtoy

### Operation

Adjust the parameters. Proceed to the direct compression of the powder.

### STEP 5: Preparation of pre-coating solution

### Equipment

High shear mixer

### Operation

Prepare the pre-coating solution by dissolving povidone into anhydrous absolute ethanol.

### STEP 6: Pre-Coating

### Equipment

Pan coating type Pelligrini

### Operation

The tablets are coated

### STEP 7: Preparation of Enteric coating suspension or solution

### Equipment

High shear mixer

### Operation

Prepare the coating suspension by suspending Hypromellose phthalate in a mixture ethanol-water (85 / 15 w/w).
Stirring constantly with a high shear mixer equipment and add triacetin, talc and red iron oxide. Homogenize.

### STEP 8: Coating

### Equipment

Pan coating type Pelligrini

### Operation

The tablets are coated

### STEP 9: Drying

Dry coated tablets

### STEP 10: Packaging

A part of the tablets is packaged in alu-alu blisters (stability studies).

Another part is packaged in HDPE bottles (stability studies and clinical trials).

Another possible advantage of the tablets of the present invention is the low cost of the manufacturing process, in comparison to the existing marketed compositions of omeprazole (pellets, multiple unit tabletted dosage forms).

A disintegration test has been performed to prove that the enteric coating was able to protect the composition at pH=1 for 2 hours. This test has been performed as described in E.P. 3rd edition, 2.9.1. The test has been performed on three consecutive pilot batches (R210, R211, R212/B). The results were conform to the specification for each batch since absolutely no disintegration appears on any tablets after 2 hours at pH=1.

The dissolution test has also been performed on the batch 24G00/B and meets the specification (not less than 80% of omeprazole dissolved 60 minutes after starting the dissolution test). The dissolution profile of the enteric coated tablets described in this invention has been compared with the dissolution profile of various marketed forms of omeprazole: LOSEC 20 mg (Astra, Belgium), MOPRAL 20 mg (Astra, France), ANTRA MUPS 20 mg (Astra, Germany). Figure 1 gives the comparative dissolution profiles of omeprazole formulation of the invention (tablet SMB 20 mg), as well as of marketed formulations (Antra, Mopral and Losec).
It can be observed that the in vitro dissolution rates of marketed pellets and of the formulation of the present invention are similar.

## Claims

1. An enteric formulation containing at least one benzimidazole derivative, said formulation comprising:
- a core containing at least one benzimidazole derivative and at least one lipophilic antioxidant selected from the group consisting of lipophilic derivatives of ascorbic acid, lipophilic derivatives of vitamin E (α-tocopherol), BHA, BHT, Propylgallate, lipoïc acid and mixtures thereof, and
- an enteric envelope protecting the core at a pH value below 5, said enteric envelope comprising in its dry form from 20 to 70% by weight of cellulosic polymer.

2. The formulation of claim 1, in which the lipophilic antioxidant comprises at least ascorbyl palmitate

3. The formulation of claim 1, in which the core is a tablet

4. The formulation of claim 1, in which the core is a tablet, said tablets being provided with at least one enteric coating layer forming an enteric envelope, said envelope comprising in its dry form from 30 to 60% by weight, especially about 50% by weight of cellulosic polymer.

5. The formulation of any one of the claims 1 to 5, which comprises a tablet comprising at least:
- a core containing at least said benzimidazole derivative and at least one lipophilic antioxidant;
- an enteric coating layer, and
- a pre-coating layer or insulating layer extending between the core and the enteric coating layer.

6. The formulation of anyone of the claims 1 to 5, in which the core is manufactured using a direct compression process.

7. The formulation of any one of the claims 1 to 5, in which at least a part of the lipophilic antioxidant is adsorbed on a tabletting agent or granulated with a tabletting agent.

8. The formulation of claim 7, in which the core comprises tabletting exipient covered with a layer containing at least one lipophilic antioxidant.

9. The formulation of any one of the claims 1 to 5, in which the enteric envelope or coating is substantially free of benzimidazole derivative.

10. The formulation of claim 5, in which the pre-coating layer or insulating layer is substantially free of benzimidazole derivative.

11. The formulation of any one of the claims 1 to 5, in which the core comprises at least a tabletting excipient selected among the group consisting of microcrystalline cellulose, cellulose derivatives, lactose, mannitol, mono or disaccharide, and mixtures thereof, on which at least one lipophilic antioxidant is attached.

12. The formulation of claim 5, in which the pre-coating layer or the insulating layer comprises at least a polymer selected from the group consisting of povidone, derivatives of povidone, derivatives of cellulose, and mixtures thereof.

13. The formulation of any one of the preceding claims, in which the enteric layer or envelope comprises at least hypromellose phthalate.

14. The formulation of any one of the claims 1 to 13, in which the enteric coating or envelope comprises at least an acrylic/methacrylic polymer or copolymer, preferably a methacrylic acid copolymer.

15. The formulation of any one of the preceding claims, in which the benzimidazole derivative is omeprazole.

16. The formulation of any one of the claims 1 to 14, in which the benzimidazole derivative is selected from the group consisting of benzimidazole derivatives inhibiting the proton pump, pantoprazole, lansoprazole, omeprazole, rabeprazole and mixtures thereof.

17. The formulation of any one of the preceding claims, in the form of a tablet or capsule containing from 5 to 80 mg omeprazole.

18. A process for the preparation of a formulation of anyone of the preceding claims, in which the core is prepared by direct compression from a mixture comprising one or more tabletting excipient and at least one lipophilic antioxidant derivative, and in which the core is provided with at least an enteric layer or envelope.

19. The process of anyone of the claims 18, in which the core has the form of a tablet, said tablet being provided with an enteric coating and with a pre-coating by using the pan-coating technology or the fluid bed technology.

20. Use of at least one lipophilic antioxidant selected from the group consisting of lipophilic derivatives of ascorbic acid, lipophilic derivatives of vitamin E (α-tocopherol), BHA, BHT, Propylgallate, lipoïc acid and mixtures thereof one, in the preparation of a benzimidazole containing core.

## Patentansprüche

1. Enterische Zubereitung, die mindestens ein Benzimidazol-Derivat enthält, welche Zubereitung aufweist:
- einem Kern, der mindestens ein Benzimidazol-Derivat und mindestens ein lipophiles Antioxidans enthält, das aus der Gruppe ausgewählt ist, die aus lipophilen Derivaten von Ascorbinsäure, lipophilen Derivaten von Vitamin E (α-Tocopherol), BHA, BHT, Propylgallat, Liponsäure und Mischungen daraus besteht, und
- einer enterischen Umhüllung, die den Kern bei einem pH-Wert unter 5 schützt, wobei die dünndarmlösliche Umhüllung in trockener Form zu 20 bis 70 Gewichts-% aus Zellulose-Polymer besteht.

2. Zubereitung nach Anspruch 1, bei der das lipophile Antioxidans zumindest aus Ascorbylpalmitat besteht.

3. Zubereitung nach Anspruch 1, bei der der Kern eine Tablette ist.

4. Zubereitung nach Anspruch 1, bei der der Kern eine Tablette ist, besagte Tabletten durch mindestens einen enterischen Überzug geschützt werden, der eine enterische Umhüllung bildet, und die besagte Umhüllung in trockener Form 30 bis 60 Gew.%, speziell etwa 50 Gew.% Zellulose-Polymer aufweist.

5. Zubereitung nach einem der Ansprüche 1 bis 5, die eine Tablette aufweist, die mindestens umfasst:
- einen Kern, der mindestens das besagte Benzimidazol-Derivat und mindestens ein lipophiles Antioxidans enthält;
- eine enterische Beschichtung, und
- eine Vorbeschichtung oder Isolierschicht, die sich zwischen Kern und enterischer Beschichtung befindet.

6. Zubereitung nach einem der Ansprüche 1 bis 5, bei der der Kern unter Verwendung eines Direktpressverfahrens hergestellt ist.

7. Zubereitung nach einem der Ansprüche 1 bis 5, bei der mindestens ein Teil des lipophilen Antioxidans an ein Tablettiermittel adsorbiert oder mit einem Tablettiermittel granuliert ist.

8. Zubereitung nach Anspruch 7, bei der der Kern ein tablettierten Arzneimittelträger aufweist, der mit einer Beschichtung versehen ist, die mindestens ein lipophiles Antioxidans enthält.

9. Zubereitung nach einem der Ansprüche 1 bis 5, bei der die enterische Umhüllung oder Beschichtung substantiell kein Benzimidazol-Derivat enthält.

10. Zubereitung nach Anspruch 5, bei der die Vorbeschichtung oder Isolierschicht substantiell kein Benzimidazol-Derivat enthält.

11. Zubereitung nach einem der Ansprüche 1 bis 5, bei der der Kern mindestens einen tablettierten Arzneimittelträger aufweist, der aus der Gruppe ausgewählt ist, die aus mikrokristalliner Zellulose, Zellulose-Derivaten, Laktose, Mannit, Mono- oder Disaccharid und Mischungen daraus besteht, an die mindestens ein lipophiles Antioxidans angelagert ist.

12. Zubereitung nach Anspruch 5, bei der die Vorbeschichtung oder Isolierschicht mindestens ein Polymer aufweist, das aus der Gruppe ausgewählt ist, die aus Povidon, Povidon-Derivaten, Zellulose-Derivaten und Mischungen daraus besteht.

13. Zubereitung nach einem der vorstehenden Ansprüche, bei der die enterische Beschichtung oder Umhüllung zumindest aus Hypromellose-phthalat besteht.

14. Zubereitung nach einem der Ansprüche 1 bis 13, bei der die enterische Beschichtung oder Umhüllung aus mindestens einem Acryl/Methacryl-Polymer oder -Copolymer, vorzugsweise einem Methacrylsäure-Copolymer besteht.

15. Zubereitung nach einem der vorstehenden Ansprüche, bei der das Benzimidazol-Derivat Omeprazol ist.

16. Zubereitung nach einem der Ansprüche 1 bis 14, bei der das Benzimidazol-Derivat aus der Gruppe ausgewählt ist, die aus Benzimidazol-Derivaten besteht, welche die Protonen-Pumpe hemmt, Pantoprazol, Lansoprazol, Omeprazol, Rabeprazol und Mischungen daraus besteht.

17. Zubereitung nach einem der vorstehenden Ansprüche in Form einer Tablette oder Kapsel, die 5 bis 80 mg Omeprazol enthält.

18. Verfahren zur Herstellung der Zubereitung nach einem der vorstehenden Ansprüche, bei dem der Kern im Direktpressverfahren aus einer Mischung hergestellt wird, die eine oder mehrere tablettierte Arzneimittelträger und mindestens ein lipophilen Antioxidans-Derivat aufweist, und in der der Kern durch mindestens eine enterische Schicht oder Umhüllung geschützt ist.

19. Verfahren nach Anspruch 18, bei dem der Kern in Form einer Tablette vorliegt, und die Tablette durch eine enterische Beschichtung und eine Vorbeschichtung geschützt ist, die mit Hilfe der Dragierkessel-Technologie oder Fließbett-Technologie aufgetragen wird.

20. Einsatz von mindestens einem lipophilen Antioxidans, das aus der Gruppe ausgewählt wird, die aus lipophilen Derivaten der Ascorbinsäure, lipophilen Derivaten von Vitamin E (α-Tocopherol), BHA, BHT, Propylgallat, Liponsäure und Mischungen daraus besteht, bei der Herstellung eines Benzimidazol enthaltenden Kerns.

## Revendications

1. Formulation entérique contenant au moins un dérivé de benzimidazole, ladite formulation comprenant :
- un noyau contenant au moins un dérivé de benzimidazole et au moins un antioxydant lipophile choisi parmi le groupe constitué de dérivés lipophiles de l'acide ascorbique, de dérivés lipophiles de la vitamine E (α-tocophérol), de BHA, de BHT, de gallate de propyle, d'acide lipoïque et des mélanges de ceux-ci, et
- une enveloppe entérique protégeant le noyau à une valeur de pH inférieure à 5, ladite enveloppe entérique comprenant dans sa forme sèche de 20 à 70% en poids de polymère cellulosique.

2. Formulation selon la revendication 1, dans laquelle l'antioxydant lipophile comprend au moins du palmitate d'ascorbyle.

3. Formulation selon la revendication 1, dans laquelle le noyau est un comprimé.

4. Formulation selon la revendication 1 dans laquelle le noyau est un comprimé, lesdits comprimés étant dotés d'au moins une couche d'enrobage entérique formant une enveloppe entérique, ladite enveloppe comprenant dans sa forme sèche de 30 à 60% en poids, spécialement environ 50% en poids de polymère cellulosique.

5. Formulation selon l'une quelconque des revendications 1 à 5, qui comprend un comprimé comprenant au moins :
- un noyau contenant au moins ledit dérivé de benzimidazole et au moins un antioxydant lipophile ;
- une couche d'enrobage entérique, et
- une couche de pré-enrobage ou couche isolante s'étendant entre le noyau et la couche d'enrobage entérique.

6. Formulation selon l'une quelconque des revendications 1 à 5, dans laquelle le noyau est fabriqué en utilisant un procédé de compression directe.

7. Formulation selon l'une quelconque des revendications 1 à 5, dans laquelle au moins une partie de l'antioxydant lipophile est adsorbée sur un agent pour la fabrication des comprimés ou granulée avec un agent pour la fabrication des comprimés.

8. Formulation selon la revendication 7, dans laquelle le noyau comprend l'excipient pour la fabrication des comprimés couvert d'une couche contenant au moins un antioxydant lipophile.

9. Formulation selon l'une quelconque des revendications 1 à 5, dans laquelle l'enveloppe ou l'enrobage entérique est substantiellement exempt de dérivé de benzimidazole.

10. Formulation selon la revendication 5, dans laquelle la couche de pré-enrobage ou couche isolante est substantiellement exempte de dérivé de benzimidazole.

11. Formulation selon l'une quelconque des revendications 1 à 5, dans laquelle le noyau comprend au moins un excipient pour la fabrication des comprimés choisi parmi le groupe constitué de cellulose microcristalline, de dérivés de cellulose, de lactose, de mannitol, de mono et disaccharide et des mélanges de ceux-ci, sur lequel au moins un antioxydant lipophile est attaché.

12. Formulation selon la revendication 5, dans laquelle la couche de pré-enrobage ou la couche isolante comprend au moins un polymère choisi parmi le groupe constitué de la povidone, des dérivés de povidone, des dérivés de cellulose et des mélanges de ceux-ci.

13. Formulation selon l'une quelconque des revendications précédentes, dans laquelle la couche ou enveloppe entérique comprend au moins le phtalate d'hypromellose.

14. Formulation selon l'une quelconque des revendications 1 à 13, dans laquelle l'enrobage ou enveloppe entérique comprend au moins un polymère ou copolymère acrylique/méthacrylique, de préférence un copolymère d'acide méthacrylique.

15. Formulation selon l'une quelconque des revendications précédentes, dans laquelle le dérivé de benzimidazole est l'oméprazole.

16. Formulation selon l'une quelconque des revendications 1 à 14, dans laquelle le dérivé de benzimidazole est choisi parmi le groupe constitué des dérivés de benzimidazole inhibant la pompe à protons, le pantoprazole, le lansoprazole, l'oméprazole, le rabéprazole et des mélanges de ceux-ci.

17. Formulation selon l'une quelconque des revendications précédentes, sous la forme d'un comprimé ou d'une capsule contenant de 5 à 80 mg d'oméprazole.

18. Procédé pour la préparation d'une formulation selon l'une quelconque des revendications précédentes, dans lequel le noyau est préparé par compression directe à partir d'un mélange comprenant un ou plusieurs excipients pour la fabrication des comprimés et au moins un dérivé antioxydant lipophile et dans lequel le noyau est doté d'au moins une couche ou enveloppe entérique.

19. Procédé selon l'une quelconque des revendications 1-18, dans lequel le noyau a la forme d'un comprimé, ledit comprimé étant doté d'un enrobage entérique et d'un pré-enrobage en utilisant la technologie d'enrobage en turbine ou la technologie du lit fluidisé.

20. Utilisation d'au moins un antioxydant lipophile choisi parmi le groupe constitué de dérivés lipophiles de l'acide ascorbique, de dérivés lipophiles de la vitamine E (α-tocophérol), de BHA, de BHT, de gallate de propyle, d'acide lipoïque et des mélanges de ceux-ci, dans la préparation d'un noyau contenant du benzimidazole.
